# EUROPEAN PATENT SPECIFICATION

(11) **EP 3 538 652 B1**
(45) Date of publication and mention of the grant of the patent: **10.11.2021**
(21) Application number: 16794993.2
(22) Date of filing: 08.11.2016
(51) Int. Cl.: A21D 8/04, A23L 2/66, A61K 38/00, A61K 45/06, A61P 35/02, A61P 35/00, A23L 7/104, C12N 9/82

(54) **A HIGHLY STABLE, PROTEASE-RESISTANT E. COLI ASPARAGINASE**
HOCHSTABILE PROTEASE-RESISTENTE E. COLI-ASPARAGINASE
E RÉSISTANT AUX PROTÉASES HAUTEMENT STABLE ASPARAGINASE COLI

(43) Date of publication of application: 18.09.2019
(73) Proprietor: Universita' Degli Studi Di Pavia, 27100 Pavia (IT)
(72) Inventor: MAGGI, Maristella, 27100 Pavia (IT); SCOTTI, Claudia, 27100 Pavia (IT)
(74) Representative: Croce, Valeria
(86) International application number: PCT/EP2016/076994
(87) International publication number: WO 2018/086676

(56) References cited:
- Marc N Offman ET AL: "Rational engineering of L-asparaginase reveals importance of dual activity for cancer cell toxicity", Blood, 1 January 2011 (2011-01-01), pages 1614-1621, XP055335115, DOI: 10.1182/blood-2010- Retrieved from the Internet: URL:http://www.bloodjournal.org/content/11 7/5/1614.full.pdf [retrieved on 2017-01-13]
- PARMENTIER JEAN HUGUES ET AL: "Glutaminase activity determines cytotoxicity ofl-asparaginases on most leukemia cell lines", LEUKEMIA RESEARCH, vol. 39, no. 7, 1 January 2015 (2015-01-01), pages 757-762, XP029140830, ISSN: 0145-2126, DOI: 10.1016/J.LEUKRES.2015.04.008
- SHRIVASTAVA ABHINAV ET AL: "Recent developments inl-asparaginase discovery and its potential as anticancer agent", CRITICAL REVIEWS IN ONCOLOGY/HEMATOLOGY, vol. 100, 1 April 2016 (2016-04-01), pages 1-10, XP029449789, ISSN: 1040-8428, DOI: 10.1016/J.CRITREVONC.2015.01.002

SEQUENCE LISTING
<110> Università degli Studi di Pavia
<120> A highly stable, protease-resistant E. coli
   asparaginase
<130> @E0105819
<160> 8
<170> PatentIn version 3.5
<210> 1
   <211> 48
   <212> DNA
   <213> artificial
<220>
   <223> primer
<400> 1
   caccatgcat catcatcatc atcatttacc caatatcacc attttagc
   48
<210> 2
   <211> 41
   <212> DNA
   <213> artificial
<220>
   <223> Anti-sense primer
<400> 2
   caatctcgag ttattattag tactgattga agatctgctg g
   41
<210> 3
   <211> 23
   <212> DNA
   <213> artificial
<220>
   <223> Sense primer construct N24S
<400> 3
   aaccaaatct agctacacag tgg
   23
<210> 4
   <211> 25
   <212> DNA
   <213> artificial
<220>
   <223> Anti-sense primer construct N24S
<400> 4
   ccactgtgta gctagatttg gttgc
   25
<210> 5
   <211> 978
   <212> DNA
   <213> artificial
<220>
   <223> Sequence encoding N24S
<400> 5
<210> 6
   <211> 326
   <212> PRT
   <213> artificial
<220>
   <223> Sequence N24S
<400> 6
   Leu Pro Asn Ile Thr Ile Leu Ala Thr Gly Gly Thr Ile
   Ala Gly Gly
   1               5                   10
   15
   Gly Asp Ser Ala Thr Lys Ser Ser Tyr Thr Val Gly Lys
   Val Gly Val
               20                  25                  30
   Glu Asn Leu Val Asn Ala Val Pro Gln Leu Lys Asp Ile
   Ala Asn Val
           35                  40                  45
   Lys Gly Glu Gln Val Val Asn Ile Gly Ser Gln Asp Met
   Asn Asp Asn
       50                  55                  60
   Val Trp Leu Thr Leu Ala Lys Lys Ile Asn Thr Asp Cys
   Asp Lys Thr
   65 70 75
   80
   Asp Gly Phe Val Ile Thr His Gly Thr Asp Thr Met Glu
   Glu Thr Ala
   85 90
   95
   Tyr Phe Leu Asp Leu Thr Val Lys Cys Asp Lys Pro Val
   Val Met Val
   100 105
   110
   Gly Ala Met Arg Pro Ser Thr Ser Met Ser Ala Asp Gly
   Pro Phe Asn
   115 120 125
   Leu Tyr Asn Ala Val Val Thr Ala Ala Asp Lys Ala Ser
   Ala Asn Arg
   130 135 140
   Gly Val Leu Val Val Met Asn Asp Thr Val Leu Asp Gly
   Arg Asp Val
   145 150 155
   160
   Thr Lys Thr Asn Thr Thr Asp Val Ala Thr Phe Lys Ser
   Val Asn Tyr
   165 170
   175
   Gly Pro Leu Gly Tyr Ile His Asn Gly Lys Ile Asp Tyr
   Gln Arg Thr
   180 185
   190
   Pro Ala Arg Lys His Thr Ser Asp Thr Pro Phe Asp Val
   Ser Lys Leu
   195 200 205
   Asn Glu Leu Pro Lys Val Gly Ile Val Tyr Asn Tyr Ala Asn Ala Ser
   210 215 220
   Asp Leu Pro Ala Lys Ala Leu Val Asp Ala Gly Tyr Asp Gly Ile Val
   225 230 235 240
   Ser Ala Gly Val Gly Asn Gly Asn Leu Tyr Lys Ser Val Phe Asp Thr
   245 250 255
   Leu Ala Thr Ala Ala Lys Thr Gly Thr Ala Val Val Arg Ser Ser Arg
   260 265 270
   Val Pro Thr Gly Ala Thr Thr Gln Asp Ala Glu Val Asp Asp Ala Lys
   275 280 285
   Tyr Gly Phe Val Ala Ser Gly Thr Leu Asn Pro Gln Lys Ala Arg Val
   290 295 300
   Leu Leu Gln Leu Ala Leu Thr Gln Thr Lys Asp Pro Gln Gln Ile Gln
   305 310 315 320
   Gln Ile Phe Asn Gln Tyr
   325
<210> 7
   <211> 978
   <212> DNA
   <213> Escherichia coli
<400> 7
   ttacccaata tcaccatttt agcaaccggc gggaccattg ccggtggtgg tgactccgca 60
   accaaatcta actacacagt gggtaaagtt ggcgtagaaa atctggttaa tgcggtgccg 120
   caactaaaag acattgcgaa cgttaaaggc gagcaggtag tgaatatcgg ctcccaggac 180
   atgaacgata atgtctggct gacactggcg aaaaaaatta acaccgactg cgataagacc 240
   gacggcttcg tcattaccca cggtaccgac acgatggaag aaactgctta cttcctcgac 300
   ctgacggtga aatgcgacaa accggtggtg atggtcggcg caatgcgtcc gtccacgtct 360
   atgagcgcag acggtccatt caacctgtat aacgcggtag tgaccgcagc tgataaagcc 420
   tccgccaacc gtggcgtgct ggtagtgatg aatgacaccg tgcttgatgg ccgtgacgtc 480
   accaaaacca acaccaccga cgtagcgacc ttcaagtctg ttaactacgg tcctctgggt 540
   tacattcaca acggtaagat tgactaccag cgtaccccgg cacgtaagca taccagcgac 600
   acgccattcg atgtctctaa gctgaatgaa ctgccgaaag tcggcattgt ttataactac 660
   gctaacgcat ccgatcttcc ggctaaagca ctggtagatg cgggctatga tggcatcgtt 720
   agcgctggtg tgggtaacgg caacctgtat aaatctgtgt tcgacacgct ggcgaccgcc 780
   gcgaaaaccg gtactgcagt cgtgcgttct tcccgcgtac cgacgggcgc taccactcag 840
   gatgccgaag tggatgatgc gaaatacggc ttcgtcgcct ctggcacgct gaacccgcaa 900
   aaagcgcgcg ttctgctgca actggctctg acgcaaacca aagatccgca gcagatccag 960
   cagatcttca atcagtac 978
<210> 8
   <211> 326
   <212> PRT
   <213> Escherichia coli
<400> 8
   Leu Pro Asn Ile Thr Ile Leu Ala Thr Gly Gly Thr Ile
   Ala Gly Gly
   1 5 10
   15
   Gly Asp Ser Ala Thr Lys Ser Asn Tyr Thr Val Gly Lys
   Val Gly Val
   20 25 30
   Glu Asn Leu Val Asn Ala Val Pro Gln Leu Lys Asp Ile
   Ala Asn Val
   35 40 45
   Lys Gly Glu Gln Val Val Asn Ile Gly Ser Gln Asp Met
   Asn Asp Asn
   50 55 60
   Val Trp Leu Thr Leu Ala Lys Lys Ile Asn Thr Asp Cys
   Asp Lys Thr
   65 70 75
   80
   Asp Gly Phe Val Ile Thr His Gly Thr Asp Thr Met Glu
   Glu Thr Ala
   85 90
   95
   Tyr Phe Leu Asp Leu Thr Val Lys Cys Asp Lys Pro Val
   Val Met Val
   100 105
   110
   Gly Ala Met Arg Pro Ser Thr Ser Met Ser Ala Asp Gly
   Pro Phe Asn
   115 120 125
   Leu Tyr Asn Ala Val Val Thr Ala Ala Asp Lys Ala Ser
   Ala Asn Arg
   130 135 140
   Gly Val Leu Val Val Met Asn Asp Thr Val Leu Asp Gly
   Arg Asp Val
   145 150 155
   160
   Thr Lys Thr Asn Thr Thr Asp Val Ala Thr Phe Lys Ser
   Val Asn Tyr
   165 170
   175
   Gly Pro Leu Gly Tyr Ile His Asn Gly Lys Ile Asp Tyr
   Gln Arg Thr
   180 185
   190
   Pro Ala Arg Lys His Thr Ser Asp Thr Pro Phe Asp Val
   Ser Lys Leu
   195 200 205
   Asn Glu Leu Pro Lys Val Gly Ile Val Tyr Asn Tyr Ala
   Asn Ala Ser
   210 215 220
   Asp Leu Pro Ala Lys Ala Leu Val Asp Ala Gly Tyr Asp
   Gly Ile Val
   225 230 235
   240
   Ser Ala Gly Val Gly Asn Gly Asn Leu Tyr Lys Ser Val
   Phe Asp Thr
   245 250
   255
   Leu Ala Thr Ala Ala Lys Thr Gly Thr Ala Val Val Arg
   Ser Ser Arg
   260 265
   270
   Val Pro Thr Gly Ala Thr Thr Gln Asp Ala Glu Val Asp
   Asp Ala Lys
   275 280 285
   Tyr Gly Phe Val Ala Ser Gly Thr Leu Asn Pro Gln Lys
   Ala Arg Val
       290                 295                 300
   Leu Leu Gln Leu Ala Leu Thr Gln Thr Lys Asp Pro Gln
   Gln Ile Gln
   305                 310                 315
   320
   Gln Ile Phe Asn Gln Tyr
                   325

SEQUENCE LISTING
<110> Università degli Studi di Pavia
<120> A highly stable, protease-resistant E. coli asparaginase
<130> @E0105819
<160> 8
<170> PatentIn version 3.5
<210> 1
   <211> 48
   <212> DNA
   <213> artificial
<220>
   <223> primer
<400> 1
   caccatgcat catcatcatc atcatttacc caatatcacc attttagc 48
<210> 2
   <211> 41
   <212> DNA
   <213> artificial
<220>
   <223> Anti-sense primer
<400> 2
   caatctcgag ttattattag tactgattga agatctgctg g 41
<210> 3
   <211> 23
   <212> DNA
   <213> artificial
<220>
   <223> Sense primer construct N24S
<400> 3
   aaccaaatct agctacacag tgg 23
<210> 4
   <211> 25
   <212> DNA
   <213> artificial
<220>
   <223> Anti-sense primer construct N24S
<400> 4
   ccactgtgta gctagatttg gttgc 25
<210> 5
   <211> 978
   <212> DNA
   <213> artificial
<220>
   <223> Sequence encoding N24S
<400> 5
   ttacccaata tcaccatttt agcaaccggc gggaccattg ccggtggtgg tgactccgca      60
   accaaatcta gctacacagt gggtaaagtt ggcgtagaaa atctggttaa tgcggtgccg     120
   caactaaaag acattgcgaa cgttaaaggc gagcaggtag tgaatatcgg ctcccaggac     180
   atgaacgata atgtctggct gacactggcg aaaaaaatta acaccgactg cgataagacc     240
   gacggcttcg tcattaccca cggtaccgac acgatggaag aaactgctta cttcctcgac     300
   ctgacggtga aatgcgacaa accggtggtg atggtcggcg caatgcgtcc gtccacgtct     360
   atgagcgcag acggtccatt caacctgtat aacgcggtag tgaccgcagc tgataaagcc     420
   tccgccaacc gtggcgtgct ggtagtgatg aatgacaccg tgcttgatgg ccgtgacgtc     480
   accaaaacca acaccaccga cgtagcgacc ttcaagtctg ttaactacgg tcctctgggt     540
   tacattcaca acggtaagat tgactaccag cgtaccccgg cacgtaagca taccagcgac     600
   acgccattcg atgtctctaa gctgaatgaa ctgccgaaag tcggcattgt ttataactac     660
   gctaacgcat ccgatcttcc ggctaaagca ctggtagatg cgggctatga tggcatcgtt     720
   agcgctggtg tgggtaacgg caacctgtat aaatctgtgt tcgacacgct ggcgaccgcc     780
   gcgaaaaccg gtactgcagt cgtgcgttct tcccgcgtac cgacgggcgc taccactcag     840
   gatgccgaag tggatgatgc gaaatacggc ttcgtcgcct ctggcacgct gaacccgcaa     900
   aaagcgcgcg ttctgctgca actggctctg acgcaaacca aagatccgca gcagatccag     960
   cagatcttca atcagtac                                                   978
<210> 6
   <211> 326
   <212> PRT
   <213> artificial
<220>
   <223> Sequence N24S
<400> 6
   Leu Pro Asn Ile Thr Ile Leu Ala Thr Gly Gly Thr Ile Ala Gly Gly
   1               5                   10                  15
   Gly Asp Ser Ala Thr Lys Ser Ser Tyr Thr Val Gly Lys Val Gly Val
               20                  25                  30
   Glu Asn Leu Val Asn Ala Val Pro Gln Leu Lys Asp Ile Ala Asn Val
           35                  40                  45
   Lys Gly Glu Gln Val Val Asn Ile Gly Ser Gln Asp Met Asn Asp Asn
       50                  55                  60
   Val Trp Leu Thr Leu Ala Lys Lys Ile Asn Thr Asp Cys Asp Lys Thr
   65                  70                  75                  80
   Asp Gly Phe Val Ile Thr His Gly Thr Asp Thr Met Glu Glu Thr Ala
                   85                  90                  95
   Tyr Phe Leu Asp Leu Thr Val Lys Cys Asp Lys Pro Val Val Met Val
               100                 105                 110
   Gly Ala Met Arg Pro Ser Thr Ser Met Ser Ala Asp Gly Pro Phe Asn
           115                 120                 125
   Leu Tyr Asn Ala Val Val Thr Ala Ala Asp Lys Ala Ser Ala Asn Arg
       130                 135                 140
   Gly Val Leu Val Val Met Asn Asp Thr Val Leu Asp Gly Arg Asp Val
   145                 150                 155                 160
   Thr Lys Thr Asn Thr Thr Asp Val Ala Thr Phe Lys Ser Val Asn Tyr
                   165                 170                 175
   Gly Pro Leu Gly Tyr Ile His Asn Gly Lys Ile Asp Tyr Gln Arg Thr
               180                 185                 190
   Pro Ala Arg Lys His Thr Ser Asp Thr Pro Phe Asp Val Ser Lys Leu
           195                 200                 205
   Asn Glu Leu Pro Lys Val Gly Ile Val Tyr Asn Tyr Ala Asn Ala Ser
       210                 215                 220
   Asp Leu Pro Ala Lys Ala Leu Val Asp Ala Gly Tyr Asp Gly Ile Val
   225                 230                 235                 240
   Ser Ala Gly Val Gly Asn Gly Asn Leu Tyr Lys Ser Val Phe Asp Thr
                   245                 250                 255
   Leu Ala Thr Ala Ala Lys Thr Gly Thr Ala Val Val Arg Ser Ser Arg
               260                 265                 270
   Val Pro Thr Gly Ala Thr Thr Gln Asp Ala Glu Val Asp Asp Ala Lys
           275                 280                 285
   Tyr Gly Phe Val Ala Ser Gly Thr Leu Asn Pro Gln Lys Ala Arg Val
       290                 295                 300
   Leu Leu Gln Leu Ala Leu Thr Gln Thr Lys Asp Pro Gln Gln Ile Gln
   305                 310                 315                 320
   Gln Ile Phe Asn Gln Tyr
                   325
<210> 7
   <211> 978
   <212> DNA
   <213> Escherichia coli
<400> 7
   ttacccaata tcaccatttt agcaaccggc gggaccattg ccggtggtgg tgactccgca      60
   accaaatcta actacacagt gggtaaagtt ggcgtagaaa atctggttaa tgcggtgccg     120
   caactaaaag acattgcgaa cgttaaaggc gagcaggtag tgaatatcgg ctcccaggac     180
   atgaacgata atgtctggct gacactggcg aaaaaaatta acaccgactg cgataagacc     240
   gacggcttcg tcattaccca cggtaccgac acgatggaag aaactgctta cttcctcgac     300
   ctgacggtga aatgcgacaa accggtggtg atggtcggcg caatgcgtcc gtccacgtct     360
   atgagcgcag acggtccatt caacctgtat aacgcggtag tgaccgcagc tgataaagcc     420
   tccgccaacc gtggcgtgct ggtagtgatg aatgacaccg tgcttgatgg ccgtgacgtc     480
   accaaaacca acaccaccga cgtagcgacc ttcaagtctg ttaactacgg tcctctgggt     540
   tacattcaca acggtaagat tgactaccag cgtaccccgg cacgtaagca taccagcgac     600
   acgccattcg atgtctctaa gctgaatgaa ctgccgaaag tcggcattgt ttataactac     660
   gctaacgcat ccgatcttcc ggctaaagca ctggtagatg cgggctatga tggcatcgtt     720
   agcgctggtg tgggtaacgg caacctgtat aaatctgtgt tcgacacgct ggcgaccgcc     780
   gcgaaaaccg gtactgcagt cgtgcgttct tcccgcgtac cgacgggcgc taccactcag     840
   gatgccgaag tggatgatgc gaaatacggc ttcgtcgcct ctggcacgct gaacccgcaa     900
   aaagcgcgcg ttctgctgca actggctctg acgcaaacca aagatccgca gcagatccag     960
   cagatcttca atcagtac                                                   978
<210> 8
   <211> 326
   <212> PRT
   <213> Escherichia coli
<400> 8
   Leu Pro Asn Ile Thr Ile Leu Ala Thr Gly Gly Thr Ile Ala Gly Gly
   1               5                   10                  15
   Gly Asp Ser Ala Thr Lys Ser Asn Tyr Thr Val Gly Lys Val Gly Val
               20                  25                  30
   Glu Asn Leu Val Asn Ala Val Pro Gln Leu Lys Asp Ile Ala Asn Val
           35                  40                  45
   Lys Gly Glu Gln Val Val Asn Ile Gly Ser Gln Asp Met Asn Asp Asn
       50                  55                  60
   Val Trp Leu Thr Leu Ala Lys Lys Ile Asn Thr Asp Cys Asp Lys Thr
   65                  70                  75                  80
   Asp Gly Phe Val Ile Thr His Gly Thr Asp Thr Met Glu Glu Thr Ala
                   85                  90                  95
   Tyr Phe Leu Asp Leu Thr Val Lys Cys Asp Lys Pro Val Val Met Val
               100                 105                 110
   Gly Ala Met Arg Pro Ser Thr Ser Met Ser Ala Asp Gly Pro Phe Asn
           115                 120                 125
   Leu Tyr Asn Ala Val Val Thr Ala Ala Asp Lys Ala Ser Ala Asn Arg
       130                 135                 140
   Gly Val Leu Val Val Met Asn Asp Thr Val Leu Asp Gly Arg Asp Val
   145                 150                 155                 160
   Thr Lys Thr Asn Thr Thr Asp Val Ala Thr Phe Lys Ser Val Asn Tyr
                   165                 170                 175
   Gly Pro Leu Gly Tyr Ile His Asn Gly Lys Ile Asp Tyr Gln Arg Thr
               180                 185                 190
   Pro Ala Arg Lys His Thr Ser Asp Thr Pro Phe Asp Val Ser Lys Leu
           195                 200                 205
   Asn Glu Leu Pro Lys Val Gly Ile Val Tyr Asn Tyr Ala Asn Ala Ser
       210                 215                 220
   Asp Leu Pro Ala Lys Ala Leu Val Asp Ala Gly Tyr Asp Gly Ile Val
   225                 230                 235                 240
   Ser Ala Gly Val Gly Asn Gly Asn Leu Tyr Lys Ser Val Phe Asp Thr
                   245                 250                 255
   Leu Ala Thr Ala Ala Lys Thr Gly Thr Ala Val Val Arg Ser Ser Arg
               260                 265                 270
   Val Pro Thr Gly Ala Thr Thr Gln Asp Ala Glu Val Asp Asp Ala Lys
               275                 280                 285
   Tyr Gly Phe Val Ala Ser Gly Thr Leu Asn Pro Gln Lys Ala Arg Val
       290                 295                 300
   Leu Leu Gln Leu Ala Leu Thr Gln Thr Lys Asp Pro Gln Gln Ile Gln
   305                 310                 315                 320
   Gln Ile Phe Asn Gln Tyr
                   325

## Description

### Field of the invention

The present invention finds application in the medical field and in particular for the treatment of tumors and, more specifically, for the treatment of Acute Lymphoblastic Leukaemia (ALL).

### Background

Bacterial type II L-Asparaginases (ASNases) are amidohydrolases (EC 3.5.1.1) that catalyze L-asparagine (L-Asn) hydrolysis to L-aspartic acid (L-Asp) and ammonia.

With reduced efficiency, ASNases are also capable of deaminating L-glutamine (L-Gln) releasing L-glutamic acid (L-Glu) and ammonia.

Structurally, ASNases are homotetramers of roughly 130-140 kDa.

An ASNase monomer has two well distinct domains, the N-domain (roughly 230 residues) and the C-domain (roughly 110 residues) connected by an ordered loop.

In the tetrameric fold, each monomer acquires two sets of contacts: one, less extended, that represents the distant dimer, and one, more extended, that represents the intimate dimer.

The latter dimer is the functional unit of ASNases and each of the two intimate dimers has two active sites at the subunit interface. Each active site is built up of residues belonging mainly to each monomer N-domain and, to a lower extent, to the C-domain of a second, cognate monomer.

The N-domain contains the active site flexible loop (residues 3-27), a peculiar structural element that acts as a lid that closes onto the active site upon substrate binding bringing with it two essential catalytic residues (a Thr and a Tyr).

ASNases catalytic residues are described by two highly conserved triads (Thr-Asp-Lys and Thr-Tyr-Glu) in which threonines play the main role [1].

L-Asparaginases have been isolated and described as antiproliferative agents early in the 1950s [2] and, since then, bacteria have been used as a primary source of the enzyme drug. ASNases antiproliferative mechanism relies on the prompt removal of L-asparagine from blood and affects mainly tumor cells that are often impaired in the L-Asn *de novo* biosynthetic pathway.

As a consequence of L-Asn starvation and in the absence of *de novo* production, tumor cells activate pro-apoptotic and autophagy pathways [3].

Until a few years ago, the secondary L-glutaminase activity of ASNases has been considered less relevant for the drug toxicity, and even deleterious, as L-Gln removal was considered the main cause of ASNase toxicity on healthy tissues. Nowadays, the role of L-Gln removal in ASNase anti-tumor activity has been re-evaluated and more and more evidences supporting the need of the enzyme dual activity have been reported in the literature [4-6]. L-Asparaginases have been used as first line drugs for pediatric Acute Lymphoblastic Leukemia (ALL) treatment for more than 40 years now.

Their great efficacy and peculiar starving activity make them of great interest not only for improvement of ALL therapy but also for establishment of new and more effective treatment of other tumors. Unfortunately, ASNase-based therapy has some limitations that are mainly related to its: (i) metabolic side effects, (ii) high immunogenicity, (iii) low efficacy in adult patients, and (iv) *in vivo* high instability.

In pediatric patients, early development of anti-ASNase antibodies is linked to high likelihood of therapy failure and, in turn, to a negative outcome for the patient.

Moreover, ASNase immunogenicity is the main limit for its use in adult patients since the adult immune system is more reactive to exogenous molecules than the children's one.

The approach generating anti-ASNase antibodies is not fully successful since they may cause allergic reactions, more frequently mild than severe, or silent inactivation of the drug.

The latter phenomenon is quite dangerous as it causes early inactivation of the drug without any symptom or clear manifestation that can indicate the lack of pharmacological efficacy.

Presently, *Escherichia coli* (EcAII) and *Erwinia chrysanthemi* (ErAII) type II ASNases are available for clinical use.

EcAII, both in native or PEGylated form, is used as a first choice drug; instead, ErAII is used only in case of immune reactions to E. coli-derived ASNases.

Unfortunately, both proteins are characterized by high *in vivo* instability, short half-life and the need of several administrations to obtain a pharmacological active concentration. Moreover, the protein instability is related to an increased likelihood of antibodies production and, in turn, to immune reactions.

Another aspect to be considered for ASNases clinical application is the molecule susceptibility to protease-mediated degradation.

In 2009, Patel *et al.* [7] described two lysosomal proteases capable of inactivating EcAII by hydrolysis. The two proteases are cathepsin B (CTSB) and asparagine endopeptidase (AEP) and can be over-expressed by leukaemia lymphoblasts impairing drug activity and pharmacokinetics. Moreover, also ErAII resulted to be sensitive to CTSB proteolytic degradation.

High expression of AEP and CTSB has been reported for instance in patients affected by Philadelphia positive (Ph+) and iAMP21 leukaemia, which are very aggressive and poorly responsive forms of leukemia.

In the attempt to prevent ASNases immunogenicity and at the same time to improve the molecule stability, native EcAII and ErAII proteins have been chemically modified by PEGylation.

PEGylated EcAII (PEGASPASE) and, only recently, PEGylated ErAII (namely, PEGCRISANTASPASE) are routinely used in the clinics.

Nevertheless, PEGASPASE can cause allergic reactions, even though with reduced frequency compared to the native EcAII, and in addition it can cause hypertriglyceridemia that can result in severe pancreatitis and metabolic dysfunctions.

Moreover, PEGASPASE has the same susceptibility to CTSB hydrolysis of the native enzyme, preventing its use for the treatment of ASNase-inactivating tumors.

As an alternative to PEGylated asparaginases, a native form of EcAII loaded into patients derived red blood cells (GRASPA) has recently been introduced in the clinics (Phase II/III clinical trial). The new drug delivery system seems to be quite successful in reducing ASNase immunogenicity and in improving its half-life and can be a valid new platform for safer ASNase delivery not only in pediatric patients but also in adult ones.

Thought the above, it is felt the need for further asparaginases characterized by improved thermal and proteolytic resistance.

### Summary of the invention

The present invention is based on the finding that a variant of *Escherichia coli* type II L-asparaginase presents particularly desirable properties that make it suitable for an improved ASNase-based therapy for tumors.

### Brief disclosure of the figures

Figure 1 shows the results of the tests for protein stability;

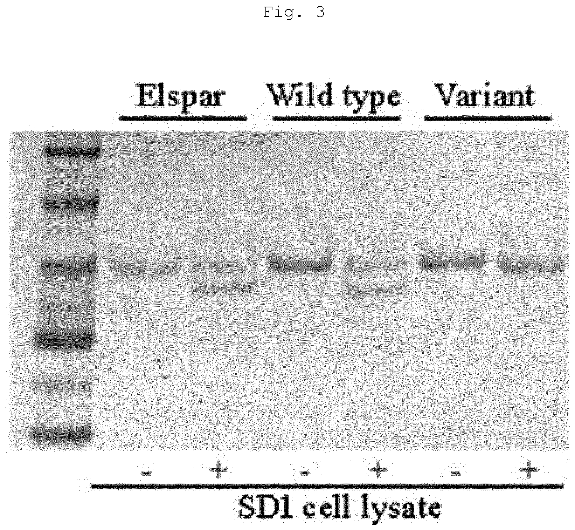

figure 2 shows the results of the thermal shift assay; figure 3 reports the results of the effect of the wild-type and N24S ASNase on SD1 (graph A) and REH cells (graph B);
figure 4 shows EcAII wild type, N24S and Elspar degradation by SD1 cell lysate (Ponceau Red staining); figure 5 reports the degradation pattern at 4 weeks of the wild-type and N24S mutant;
figure 6 illustrates the structure of the loop containing the N24 residue and the S24 residue in the wild-type and mutant proteins, respectively.

### Object of the invention

According to a first object, the present invention discloses a variant of the *Escherichia coli* type II asparaginase, which is characterized by having the sequence of SEQ. ID. N.6.

In a further object, it is disclosed a process for the preparation of the protein of the invention.

According to a second object, the asparaginase enzyme of the invention is disclosed as a medicament and, particularly, as a medicament for the treatment of tumors. The references to methods of treatment in the description are to be interpreted as references to the compounds, pharmaceutical compositions and medicaments of the present invention for use in a method for treatment of the human (or animal) body by therapy (or for diagnosis)."

The use as a medicament is intended for humans and for animals.

A further object of the invention is represented by a method for the treatment of tumors comprising the step of administering a suitable amount of the disclosed *Escherichia coli* type II asparaginase to a patient in need thereof.

The method of treatment is intended for humans and for animals.

A pharmaceutical preparation comprising the protein of the invention represents a further object of the invention.

### Detailed description of the invention

According to a first object, it is disclosed a variant of a *Escherichia coli* type II asparaginase (N24S mutant), which is characterized by having the amino acid sequence of SEQ. ID. N.6:

| | |
|---|---|
| **SEQ. ID. n. 6** | |

In particular, the protein is encoded by the nucleic acid sequence corresponding to SEQ. ID. n. 5:

| | |
|---|---|
| | |
| **SEQ. ID. n. 5** | |

According to a particular embodiment of the invention, the disclosed protein may be chemically modified with a suitable polymer.

Preferably, said polymer is represented by polyethylenglycol (PEG).

For the purposes of the invention, asparaginase PEGylation is obtained by covalent conjunction of monomethoxypolyethylene glycol (PEG, molecular weight less than or equal to 5000 Da) to native protein [8]. Bioconjugation is obtained by mixing the native protein with chemically activated PEG that binds to specific residues on the protein surface.

In an alternative embodiment, the N24S protein of the invention may be encapsulated within red blood cells. In particular, reference is made to the well-known GRASPA technology.

Encapsulation in red blood cells (RBCs) is a well-known method for safer administration of circulating drugs [9]. A commercial form of L-asparaginase encapsulated into RBCs has became recently available in the clinics (GRASPA by Eritech). The technique of encapsulation consists of a transient osmotic shock of RBCs derived from patient blood. RBCs osmotic permeation is done by gradual dialysis and causes transient permeability of cell membrane due to inflating and pores opening. Inflated RBCs are permeable to L-asparaginase that uses the opened pores to enter the cells. Subsequently, cells are returned to isotonicity and L-asparaginase persists in the cells as an active molecule [10]. Encapsulated RBCs are transfused into the patients and, upon haemolysis, L-asparaginase is released into the patient serum. RBCs used as bioreactors for L-asparaginase delivery allow for improved stability of the drug as well as reduced immunogenicity due to brief permanence of the free drug in the patient circulatory system.

In a further object, it is disclosed a process for the preparation of the protein of the invention.

In particular, the preparation of the protein of the invention comprises the step of culturing suitable host cells, which have been transformed with a vector comprising the encoding sequence corresponding to SEQ. ID. n. 5 in an appropriate medium, followed by the recovery of the extract and purification of the protein.

According to the present invention, suitable hosts may be selected in the group comprising: bacteria, yeast or mammalian cells.

In a preferred embodiment the host is represented by *Escherichia coli.*

In a more preferred embodiment, the *Escherichia coli* cell is a cell of the strain BL21(DE3).

In particular, the preparation of the protein includes the use of the sense and anti-sense primers characterized by having the following sequences:

| | |
|---|---|
| **SEQ. ID. n. 1** | |
| **SEQ. ID. n. 2** | CAATCTCGAGTTATTATTAGTACTGATTGAAGATCTGCTGG |
| **SEQ. ID. n. 3** | AACCAAATCT**AGC**TACACAGTGG |
| **SEQ. ID. n. 4** | CCACTGTGTA**GCT**AGATTTGGTTGC |

According to a second object, the asparaginase of the invention is disclosed as a medicament.

More specifically, in the following, the asparaginase of the invention is intended to be any one of the form:
- the N24S protein,
- the PEGylated N24S protein,
- the N24S protein encapsulated within red blood cells, as above disclosed.

In particular, the asparaginase finds use as a medicament for the treatment of solid tumors, both primary and secondary.

According to a particular aspect, solid tumor is used to refer to neoplasia selected in the group comprising: gastrointestinal, lung, breast, prostatic, ovarian, liver, kidney, thyroid, nasopharyngeal tumors, sarcomas, lymphomas and multiple myeloma.

Patients may be represented by adults or children.

In a preferred embodiment, the protein finds application for the treatment of Acute Lymphoblastic Leukaemia (ALL).

As a more preferred embodiment, Acute Lymphoblastic Leukaemia may be Philadelphia positive (Ph+) or iAMP21 leukaemia.

In a particular embodiment, the treatment of Acute Lymphoblastic Leukaemia is typically performed on children with an age of less than 18 years.

For the purposes of the present invention, the use as a medicament of the protein of the invention is intended not only for humans but also for animals.

A pharmaceutical preparation comprising the protein of the invention represents a further object of the invention.

In particular, the preparation comprising the protein of the invention is administered intravenously or intramuscularly.

Accordingly, a suitable pharmaceutical preparation within the purposes of the present invention includes an effective amount of the protein of the invention (N24S) and one of more carriers and/or excipients and/or preservatives acceptable from the pharmaceutical point of view.

The pharmaceutical preparation may be administered according to different posology schemes.

For the purposes of the present invention, a pharmaceutical preparation may comprise:
- the N24S protein of the invention;
- the N24S protein of the invention which has been modified by PEGylation;
- the N24S protein of the invention which has been encapsulated within red blood cells.

In particular, the N24S protein of the invention, optionally chemically modified by PEGylation is the protein above disclosed.

As for the encapsulated protein, reference is made to the well-known GRASPA technology.

A pharmaceutical preparation of red blood cells with the encapsulated N24S protein of the invention therein does represent a further object of the invention.

For the purposes of the present invention a pharmaceutical preparation may comprise, in addition to the protein of the invention in the forms above mentioned, one or more chemotherapeutic drugs.

In particular, said drug may be selected in the group comprising: aminoglutethimide, amsacrine, anastrozole, asparaginase, bcg, bicalutamide, bleomycin, bortezomib, buserelin, busulfan, camptothecin, capecitabine, carboplatin, carfilzomib, carmustine, chlorambucil, chloroquine, cisplatin, cladribine, clodronate, colchicine, cyclophosphamide, cyproterone, cytarabine, dacarbazine, dactinomycin, daunorubicin, demethoxyviridin, dichloroacetate, dienestrol, diethylstilbestrol, docetaxel, doxorubicin, epirubicin, estradiol, estramustine, etoposide, everolimus, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, lenalidomide, letrozole, leucovorin, leuprolide, levamisole, lomustine, lonidamine, mechlorethamine, medroxyprogesterone, megestrol, melphalan, mercaptopurine, mesna, metformin, methotrexate, mitomycin, mitotane, mitoxantrone, nilutamide, nocodazole, octreotide, oxaliplatin, paclitaxel, pamidronate, pemetrexed, pentostatin, perifosine, plicamycin, pomalidomide, porfimer, procarbazine, raltitrexed, rituximab, sorafenib, streptozocin, sunitinib, suramin, tamoxifen, temozolomide, temsirolimus, teniposide, testosterone, thalidomide, thioguanine, thiotepa, titanocene dichloride, topotecan, trastuzumab, tretinoin, vinblastine, vincristine, vindesine, or vinorelbine.

In the following experimental section, the protein of the invention is referred to as N24S mutant.

Also, one Unit of L-Asparaginase is defined as the amount of enzyme needed to convert 1 pmole of substrate into the product in the described conditions.

### MATERIALS AND METHODS

### 1. Mutation rationale

An N24S mutant has been designed in order to obtain an improved protein.

### 2. Cloning

The nucleotide sequence of the *ansB* gene from *Escherichia coli* strain K-12 substrain MG1655 was obtained from the NCBI Nucleotide database (NCBI Reference sequence: NC_000913.2). The strain was chosen according to Jennings and Beacham [11]. The sequence contains a 63 nucleotides-long leader sequence for the secretory signaling (leader sequence, LS) that was not included in the cloned insert. The *ansB* construct, containing 18 codons encoding for 6xHis at its 5', was obtained by PCR using a synthetic version of the full-length gene (pMA EcAnsB, GeneArt) as a template. The reaction mixture contained: 1X reaction buffer (Invitrogen), 300 µM dNTPs (Sigma-Aldrich), 100 ng template (pMA EcAnsB), 300 nM primers (Sigma-Aldrich, Table 1, wherein the underlined letters indicate extra-nucleotides not present in the original sequence), 2.5 U Platinum Pfx DNA polymerase (Invitrogen). The reaction volume was 50 µl. The obtained amplification product was used for cloning of the *ansB* gene in the expression vector.

The vector containing the *ansB* gene without LS and with the N-terminus 6xHis tag was used as a template for site-directed mutagenesis. The primers used to produce the N24S mutant are reported in Table 2, wherein the bold letters indicate the mutated codon. Underlined letters indicate replaced nucleotides. The PCR reaction mix contained: 1X reaction buffer (Agilent), 300 µM dNTPs (Sigma-Aldrich), 100 ng template, 300 nM primers (Sigma-Aldrich), 2.5 U Pfu Ultra II® DNA polymerase (Agilent). The reaction volume was 50 µl.

**Table 1**

| |
|---|
| **SENSE PRIMER** |
| CACCATGCATCATCATCATCATCATTTACCCAATATCACCATTTTAGC |
| **ANTI-SENSE PRIMER** |
| CAATCTCGAGTTATTATTAGTACTGATTGAAGATCTGCTGG |

**Table 2**

| **CONSTRUCT** N24S | |
|---|---|
| **SENSE PRIMER** | AACCAAATCT**AGC**TACACAGTGG |
| **ANTI-SENSE PRIMER** | CCACTGTGTA**GCT**AGATTTGGTTGC |

### 3. Protein over-expression and purification

To obtain recombinant protein over-expression, an *Escherichia coli* BL21(DE3) *ΔansA*/*ansB* strain was used. The strain was custom-engineered by Dr. Douglas Scott Merrell (University of the Uniformed Services University of the Health Sciences, Bethesda, MD, USA) to lack the *ans*A and *ans*B genes, encoding the *E. coli* endogenous type I and type II L-asparaginases, respectively. Protein over-expression was obtained by autoinduction according to Studier [12]. The cell extract was applied to a HisTrap 5 ml column equilibrated in NA buffer (50 mM Na-phosphate, 300 mM NaCl, 10 mM ImOH pH 8.0) at a 2.0 ml/min. Proteins were eluted using a step gradient (25, 50, 100, 250, 500 mM ImOH) and 5 ml fractions were collected. Fractions were checked for L-asparaginase activity and analyzed by SDS-PAGE using a 12% acrylamide/bisacrylamide gel. Fractions positive for the activity were pooled and dialyzed versus 50 mM Na-phosphate pH 7.4 using a 50 ml Desalting column (GE Healthcare) equilibrated in the same buffer. The eluted protein sample was applied to a 1 ml HiTrap Q XL column equilibrated in QA buffer (50 mM Na-phosphate pH 7.4). The flow-through fraction was collected, the bound proteins were eluted using elution buffer with high salt concentration (50 mM Na-phosphate, 1M NaCl pH 7.4) and 10 ml fractions were collected. The flow-through and the bound fractions were checked for L-asparaginase activity. An aliquot was analyzed by SDS-PAGE using a 12% acrylamide/bis-acrylamide gel. Fractions positive for the enzymatic activity and pure to homogeneity were used for the following experiments. Proteins to be used for cytotoxicity assays were dialyzed versus Phosphate Buffered Saline (PBS, 0.01 M Na/K-phosphate buffer, 0.0027 M KCl and 0.137 M NaCl, pH 7.4) using a 50 ml Desalting column. Proteins to be used for crystallization were dialyzed versus 0.03 M Tris-HCl, pH 8.0 using the same column. Samples were also analyzed by SEC on a Superose 12 HR 10/300 column (GE HEALTHCARE) equilibrated in 50 mM Na-phosphate pH 7.4, 100 mM NaCl. Prior to SEC, samples were concentrated using centrifugal filters with the appropriate cut-off (Amicon, Merck Millipore).

### 4. L-asparaginase and L-glutaminase activity assays

In order to determine the L-asparaginase and L-glutaminase activities, two methods were employed: a spectrophotometric one using the coupled reaction catalyzed by glutamic acid dehydrogenase (GADH), and a colorimetric one using Nessler's reagent.

The standard reaction mixture used to measure ammonia release by GDH-coupled reaction contained: 50 mM 4-(2-Hydroxyethyl)piperazine-1-ethanesulfonic acid (HEPES) buffer pH 7.5, 1 mM α-ketoglutarate (α-KG), 0.24 mM NADH, 20 U glutamic acid dehydrogenase and 10 mM L-Asn or 10 mM L-Gln, in a final volume of 0.5 ml. The reaction was started by adding enzyme solution (5-300 µg) and NADH consumption was monitored at 340 nm with a single-ray spectrophotometer. The standard protocol for ammonia determination using Nessler's reagent was adjusted for analysis in a 96-well plate. Briefly, a solution containing 50 mM Hepes pH 7.5, 0-10 mM L-Asn or 0-10 mM L-Gln and 5-30 µg enzyme solution was incubated at 37°C for 15 min for the L-asparaginase activity determination or for 30 min for L-glutaminase activity. The reaction was then stopped adding 0.075 M tricloracetic acid (TCA) on ice for 15 min. After centrifugation (14000 × g, 15 min), 10 µl reaction were used for the analysis with Nessler's reagent. According to the manufacturer instructions, Nessler's reagent was diluted 10-fold and 90 µl of working solution were added to 10 µl reaction in a final volume of 100 µl. In all cases, a negative control without enzyme was set up in order to determine the background noise due to spontaneous release of ammonia from the substrates.

In order to determine the amount of ammonia, a standard curve was set up using ammonium sulphate (10-0.005 mM) as ammonia source. Each molecule of ammonium sulphate releases 2 molecules of ammonia.

The kinetic parameter Kₘ was determined by Lineweaver-Burk plot using the Enzyme Kinetic Module 1.1 for Sigma Plot (SPSS Inc.).

k_{cat} or turn-over number is the number of catalytic events per second. Kₘ is the substrate concentration at which the reaction speed is half-maximal. k_{cat} / Kₘ is an index of catalytic efficiency at sub-saturating substrate concentration.

### 5. Cell lines

Human Acute Lymphoblastic Leukaemia (ALL) cells employed for the experiments are enlisted in Table 3. Experiments on MOLT-4, RS4;11, SD1 and REH cells were performed at the Laboratory of Endocrinology and Obesity - The Saban Research Institute - Children's Hospital, Los Angeles, CA, USA.

Human Acute Lymphoblastic Leukaemia cells (MOLT-4, SD1, REH, RS4;11) were grown in RPMI 1640 complete media (10% Fetal Bovine Serum, 2 mM L-glutamine, 50 µg/ml gentamicin). Cells were cultured in a humidified incubator at 37°C in the presence of 5% CO₂.

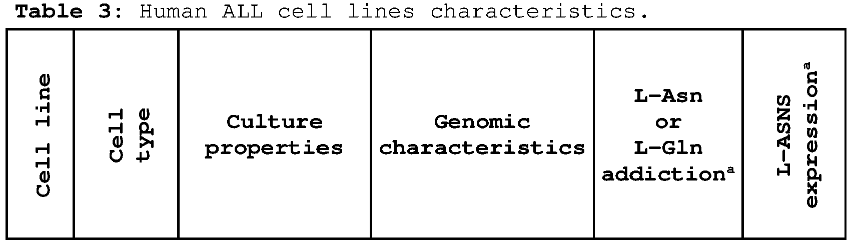

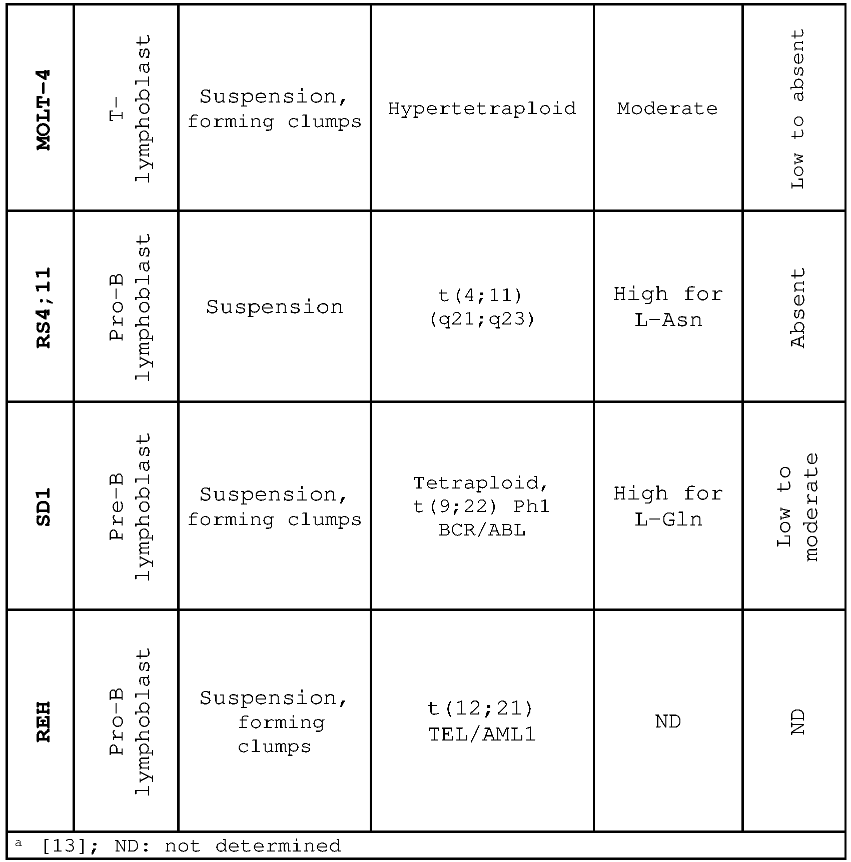

### 6. Cytotoxicity evaluation

In order to compare the cytotoxicity of recombinant wild type and N24S EcAII proteins to the one of commercial Elspar enzyme (Medac), 1 × 10⁵ cells (i.e., MOLT4, RS4;11, REH, SD1) were plated in 200 µl complete medium (RPMI 1640, 10% Fetal Bovine Serum, 2 mM L-glutamine, 50 µg/ml gentamicin). Lyophilized ASNase proteins were resuspended in complete medium and were added to the wells at a final concentration near the IC50 value for each cell line according to Parmentier *et al* [5]. Each experiment was done in triplicate and repeated at least 3 times in different days.

Trypan blue exclusion-dye count was done at 72 h by a blind observer by triturating cells in the well and by adding 2x trypan blue solution to 10 µl cell sample in a tube. Only living cells were counted.

To evaluate the improved stability of the N24S mutant lacking the main protease-recognition site, its long-term cytotoxic effect was evaluated. SD1 and REH cell lines were chosen as model systems for the study, as SD1 cells over-express proteases involved in ASNase inactivation, while REH cells do not.

1 × 10⁵ cells were plated in 1.5 ml complete medium in a 24-well plate. Cells were treated with a concentration of EcAII wild type and N24S below the IC₅₀ value for each cell line. Particularly, 0.5 U/ml and 1 U/ml were used for SD1 and REH, respectively. Cell viability was measured by trypan blue living-cell exclusion dye at 72 h and 144 h. Data were expressed as a percentage of living cells vs. initial plating (IP). Data were compared by one-way ANOVA and Tukey test. A p-value equal or lesser than 0.05 was considered significant.

### 7. Protein stability assay

Protein stability was assayed as T₅₀ value. To determine the T₅₀ of each protein, the enzyme in Na-phosphate 50 mM pH 7.4 was incubated at different temperatures (37, 40, 42, 44, 46, 48, 50, 52, 54, 56, 58, 60, 70°C) for 10 min using a thermal cycler. After incubation, the enzyme solution was cooled on ice and the enzyme activity was measured by nesslerization as previously described. The reaction mix contained 10 mM L-Asn. The T₅₀ value is determined using the Temperature Melting Plot for Sigma Plot (SPSS Inc.). The treated enzyme residual activity is expressed as percentage of heat-treated sample versus the untreated one. The T₅₀ obtained value is a good index of protein stability: the higher the T₅₀ value, the higher the protein stability.

### 8. Protease sensitivity test

Confluent SD1 cells were collected by centrifugation (3500 rpm, 5 min, 4°C) and washed twice with PBS. The washed cell pellet was resuspended in lysis buffer (50 mM trisodium acetate buffer, pH 4.5, 5 mM DTT) using a 1×10⁶ cells:0.1 ml buffer ratio. Cells were lysed by 4 cycles of freeze-thawing at -80°C. Cell lysate was clarified by centrifugation (8000 rpm, 10 min, 4°C) and whole protein extract was quantified by bicinchoninic acid assay (BCA, Pierce). The reaction mixture final volume was 50 µl and contained: 100 µg cell lysate and 4 µg ASNase (i.e., recombinant EcAII wild type and N24S, and Elspar) in 50 mM trisodium acetate buffer, pH 4.5, 5 mM DTT. After 24h incubation at 37°C, samples were added with Bolt® LDS Sample Buffer (4X, ThermoFisher Scientific) and Bolt® Sample Reducing Agent (10X, ThermoFisher Scientific) and boiled at 99°C for 5 min. Protein separation was done by electrophoresis using a precast gradient 4-12% polyacrylamide gel (Bolt® gel, ThermoFisher Scientific). Proteins were transferred onto a nitrocellulose membrane using an iBolt® 2 device (ThermoFisher Scientific). The membrane was stained using Ponceau Red dye.

### 9. Thermal shift

Thermal shift assay was conducted using a MiniOpticon real time system (Bio Rad). Samples (0.5-1.5 mg/ml) in PBS buffer were mixed with 20X SYPRO orange dye (Invitrogen) in a final volume of 50 µl in a PCR tube. Samples were centrifuged and heated from 25 to 95°C at 1°C or 0.5°C/min. Fluorescence was monitored at 517 nm. Raw data were normalized according to the following equation: fu(T) = f(T)-fn/fd-fn [14], where fu= normalized fluorescence at a given temperature (T), f= fluorescence at a given temperature (T), fn= minimum value of fluorescence, fd: maximum value of fluorescence. Accordingly, a value of 1 corresponds to completely unfolded protein. From the obtained curve, the T_{0.5} for each protein was extrapolated.

### 10. Western blot analysis

For western blot analysis, proteins were transferred from within the gel to a PVDF 0.22 µm membrane (Millipore). The blocked membrane was incubated with an HRP-conjugated anti-His tag antibody (Sigma) diluted 1:7000.

### 11. Crystallization

*Escherichia coli* type II L-asparaginase N24S mutant was purified as reported above. The pure protein fraction was dialyzed versus 30 mM Tris-HCl, pH 8.0 and used for crystallization. Crystallization attempts were carried out using a sparse-matrix screening from Molecular Dimensions (MD1-03). The crystals were grown at 21°C using the sitting-drop vapor diffusion method and by mixing equal volumes of mother liquor and 4 mg/ml protein solution in a 2 µl drop. The original crystallization condition contained 100 mM HEPES, pH 7.5, 10% w/v PEG 8000, 8% v/v ethylene glycol. In order to improve crystal quality the condition was optimized varying buffer pH and precipitants concentration. Upon cryo-mounting on nylon loops, crystals to be analyzed by X-ray were soaked in 100 µM L-aspartic acid (L-Asp) dissolved in mother liquor for 10 min.

### 12. Structure solution

X-ray data were collected at beamline ID-29 at the European Synchrotron Research Facility (ESRF), Grenoble, France [15].

The data were integrated with Mosflm [16] and merged with SCALA [17]. The space group was determined with POINTLESS [17]. The structure was solved by molecular-replacement with PHENIX-MR [18] and using an intimate dimer of L-asparaginase from the structure of *E. coli* wild-type asparaginase (PDB ID: 3ECA) [19] as a search model. Structure refinement was done with PHENIX-REFINE [18] starting from an initial model without ligand and residues belonging to the active-site flexible loop (residues 8-35). At each refinement run, residues were added manually where clear electron density was present. Ligands were added with PHENIX-LIGAND FIT and using L-aspartic acid as a search model [20] .

### RESULTS

### 1. Site directed mutagenesis

The vector containing the mutated N-terminus 6xHis tagged *Escherichia coli ansB* gene (namely, N-ansB_N24S) was sequenced for the insert (Beckman Coulter) using standard T7 primers. After sequencing, the construct with the designed mutation and devoid of any other random mutation was transformed into *E. coli* BL21(DE3) *ΔansA*/*ansB* chemically competent cells for protein over-expression.

### 2. EcAII purification

After IMAC, the N-tagged EcAII wild type and N24S protein eluted in the fractions corresponding to 250 mM ImOH together with some contaminants. Pooled and dialyzed fractions (50 mM Na-phosphate pH 7.4) were loaded onto a 1 ml HiTrap Q XL column. The protein eluted in the flow-through fraction, instead all other contaminants eluted within the bound fraction.

The wild type and N24S mutant, pure to homogeneity, had specific activity of 105±8.14 and 103.5±10.23 U/mg vs.

L-asparagine (L-Asn), respectively, and of 0.89±0.22 and 0.93±0.20 U/mg vs. L-glutamine (L-Gln), respectively. Specific activities were measured using the GADH-coupled method and using 10 mM L-Asn or L-Gln as a substrate.

The proper tetrameric fold of the protein was assessed by analytic gel-filtration using a Superose 12 HR 10/300 column equilibrated in 50 mM Na-phosphate pH 7.4. The protein eluted as a single, well-shaped peak at a volume of 13.41 ml, corresponding to roughly 136 kDa (data not shown).

### 3. EcAII wild type and N24S: kinetic characterization

EcAII wild type and the N24S variant were characterized for their kinetic characteristics versus L-Asn and L-Gln. The Nesslerization method was employed to determine the enzymes Michaelis constant (Kₘ). Kinetic parameters are reported in Table 4. Since the Nesslerization method employed to determine EcAII kinetics is not reliable for Vmax extrapolation, the k_{cat} values were calculated using the specific activity of the enzymes for each substrate.

The wild type enzyme and N24S mutant had a hyperbolic response both versus L-Asn and L-Gln. Apparent kinetic parameters obtained for the wild type protein resemble those published in the literature. The enzymes had a stronger preference for L-Asn than for L-Gln (x111 for N24S and x117 for the wild type protein) as a substrate and similar kinetic parameters.

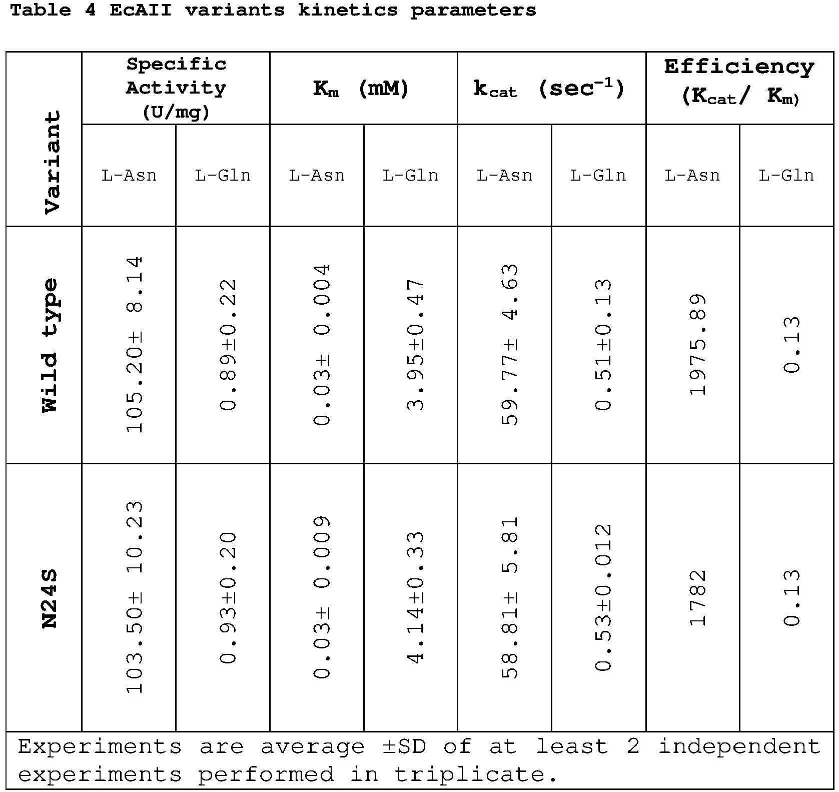

### 4. Protein stability of EcAII wild type and N24S mutant

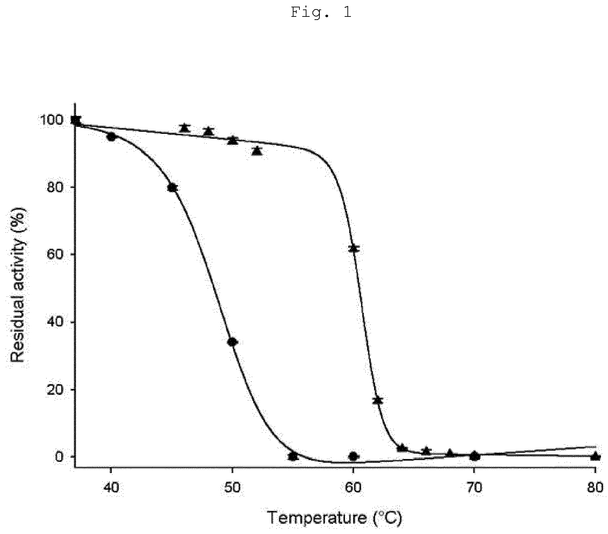

In order to evaluate the EcAII wild-type thermal stability, protein pure to homogeneity was incubated in 50 mM Na-phospate pH 7.4 at 10 different temperatures (range, 40-70°C) for 10 min. After incubation, the protein was cooled on ice and the residual activity was measured using the Nessler's method in the presence of 10 mM ASN. Residual activity was calculated as a percentage of activity of the heat-treated sample vs. the untreated protein. The melting temperature, defined as the temperature at which the activity is half of the untreated sample after 10 min incubation, was determined as T₅₀. EcAII had a T₅₀ value equal to 49±1.3 °C, the N24S mutant had a T₅₀ equal to 59±2.2 °C (Figure 1).

EcAII wild type stability at +4°C was 1-1.5 weeks, N24S mutant stability was tested at 9 weeks and 1 year and no degradation was found on an SDS gel. The time-dependent degradation of the proteins resulted in the enzyme loss of activity.

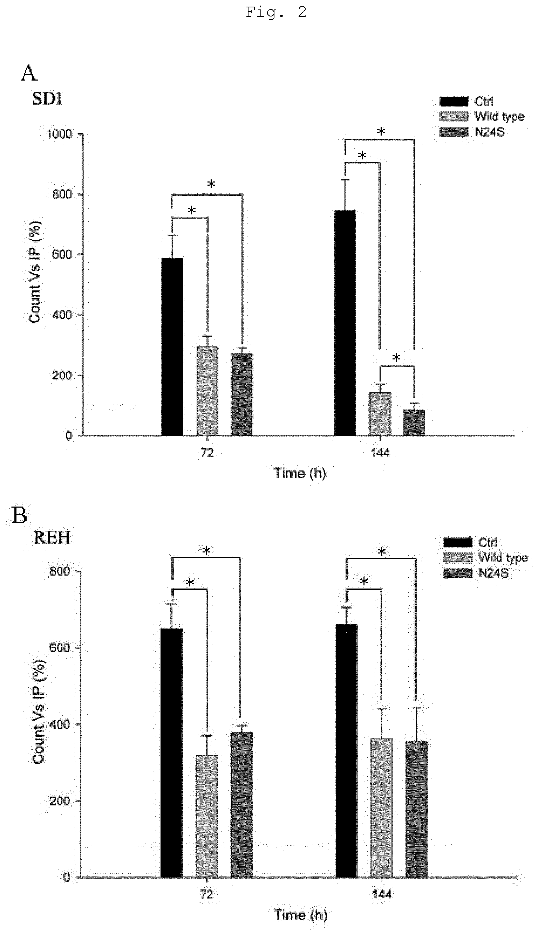

Heat-induced unfolding of EcAII wild type and N24S proteins was evaluated by thermal shift analysis using the temperature at which half of the protein was in an unfolded state (T_{0.5}) as a reference parameter. The N24S mutant had a 2.41°C higher T_{0.5} value with regard to the wild type protein (T_{0.5} 62.04±0.10°C vs. 59.63±0.62°C, respectively, Figure 2). Moreover, the wild type protein had quicker unfolding kinetics as the unfolded fraction increased from 10% to 100% in 12°C (equivalent to 7.5%/°C), whereas the N24S mutant had a slower unfolding kinetics with an unfolding rate of 5.3%/°C. Wild type initial unfolding (i.e. 5% unfolded fraction) started at 51°C, a value 6°C lower than the N24S mutant (57°C).

Enzyme stability can be evaluated by different methods. Here we analyzed the EcAII wild-type and N24S stability both as a function of biological activity retention (T₅₀ or residual activity) and as unfolding rate (T_{0.5} or Thermal shift). The former method consists of heat-treatment of the enzyme at different temperatures for a given time (i.e., 10 min), followed by immediate cooling down on ice and, finally, enzyme activity evaluation. It is a good parameter for evaluating the capability of the enzyme to maintain its functional folding when exposed to increasing temperature, consequently, higher T50 values are a good index of the enzyme functional stability. In this respect, EcAII N24S resulted to have a T₅₀ value 10°C higher than the wild type (59°C vs. 49°C, respectively), indicating that the variant is capable of maintaining its functional folding in more extreme condition than the wild type enzyme. Such a characteristic of the N24S variant is quite a promising indication of its capability of retained activity when administrated as a drug to patients. Moreover, the enzymes residual activity was evaluated upon storage at +4°C. The N24S variant displayed 8-times longer stability than the wild type protein, confirming the variant improved biological stability. The unfolding rate of wild type and N24S proteins was also evaluated by thermal shift. The T_{0.5} delta value is lower than the one obtained from residual activity evaluation (Δ, 2.41°C vs. 10°C) indicating that the N24S improved stability is mainly linked to active site stabilization than to stabilization of the tetramer. Asparaginases active site plays an essential role in protein stabilization, particularly because of the presence of the active site flexible loop (residues 3-27), a moving structural element that contains essential residues for the catalysis. The hypothesis is confirmed by the observation that protein loss of activity is proportional to the presence in SDS PAGE of a degradation band located roughly 3 kDa below the monomer molecular weight. Moreover, the lower band resulted un-reactive to an anti-His tag antibody in western blot, indicating the loss of the protein N-terminus that contains both the 6xHis tag and the active site loop.

A further confirmation derives from structural studies. The mutant N24S structure obtained by X-ray analysis resulted to be very similar to the wild type as for global folding (RMS 0.232 Å, calculated using standard pymol alignment) as well as for interface solvation energy (wild type -291.3 kcal/mol, N24S - 290.3 kcal/mol) and solvent accessible area (wild type 14366.0 Å, N24S 14148.9 Å). A significant difference between the wild type and the mutant tetrameric assembly was found in the binding (ΔGᵢₙₜ) and in the dissociation (ΔG_{diss}) energy. Particularly, the N24S ΔGᵢₙₜ was calculated to be 10.1 kcal/mol lower than the wild type one (i.e. -50.8 versus -40.7 kcal/mol, respectively), with a ΔG_{diss} variation of 4.8 kcal/mol (wild type 21.5 kcal/mol, N24S 26.3 kcal/mol). All the calculations were done using the Embl-Ebi open-access software PISA.

### 5. EcAII wild type and N24S mutant cytotoxicity

In order to compare the *in vitro* anti-proliferative effect of EcAII wild type and N24S proteins to the commercial enzyme Elspar (Medac), cytotoxicity assays were performed as described in the Materials and Methods section using 4 ALL model cell lines (i.e., MOLT-4, REH, RS4;11 and SD1). Cell viability was expressed as a percentage of viable cells with regard to the untreated sample and was assessed using EcAII wild type and N24S and Elspar concentration (U/ml) near the IC50 value described by Parmentier *et al*.[5]. Protein concentration for each cell line and percentage of viable cells at 72 h are reported in Table 5. Recombinant wild type and N24S EcAII proteins showed a cytotoxic effect comparable to the commercial Elspar protein on all cell lines but SD1. Actually, EcAII proteins had a nearly 4.30-fold higher cytotoxic effect on SD1 cells at 3.0 U/ml with respect to the commercial enzyme Elspar.

Long-term cytotoxicity was also evaluated at 72 and 144h on SD1 and REH cell lines. Wild type and N24S mutant had the same cytotoxic effect on both SD1 and REH cells at 72 h. At 144 h the EcAII variant N24S had a 47.5% higher cytotoxicity on SD1 cells than the wild type, instead both wild type and mutant had the same cytotoxicity on REH cells (figures 2A and 2B).

**Table 5 EcAII wild type, variant and Elspar cytotoxicity at 72 h**

| **Cell line** | **U/ml** | **Viability (%)** | | |
|---|---|---|---|---|
| | | **EcAII** | **N24S** | **Elspar** |
| MOLT-4 | 0.005 | 29.92±3.47 | 28.21±1.35 | 28.85±0.77 |
| REH | 3.0 | 55.21±2.80 | 61.80±6.52 | 58.8±11.55 |
| RS4;11 | 0.005 | 29.30±5.57 | 24.0.3±3.56 | 25.15±1.39 |
| SD1 | 3.0 | 19.88±6.86 | 21.72±4.96 | 84.51±8.60 |
| Experiments are average ±SD of at least 2 independent experiments performed in triplicate. | | | | |

### 6. Protease sensitivity test

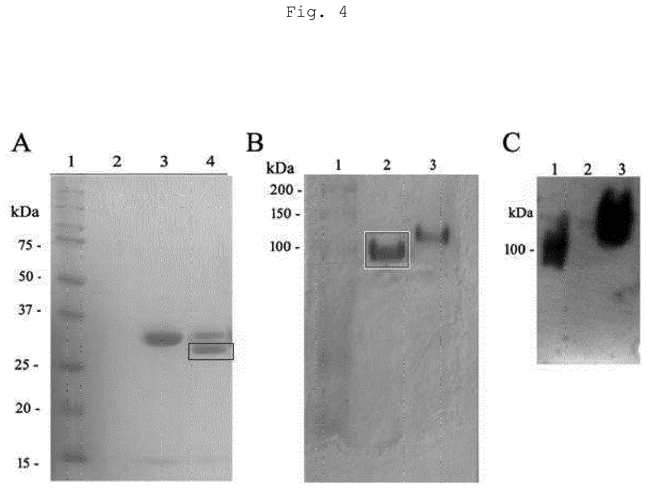

Wild type and N24S EcAII and Elspar were incubated at 37°C o.n. in the presence of SD1 cell lysate in order to determine their sensitivity to tumor-released proteases. After incubation, proteins in the samples were separated by SDS-PAGE and blotted onto a nitrocellulose membrane. After Ponceau Red staining, a clear degradation band was evident in the wild type and Elspar samples and it was absent in the N24S sample (Figure 4). The experiment was repeated 4 times, always obtaining the same result.

### 7. Stability upon storage

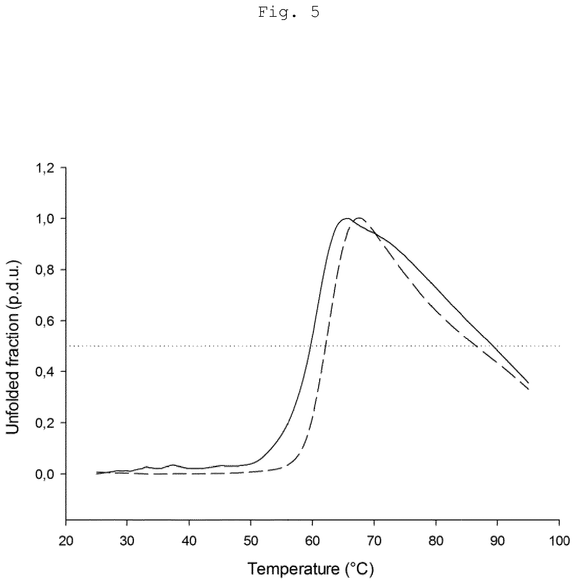

The time-dependent degradation of the EcAII wild type and N24S proteins was evaluated by electrophoretic separation and by western blot. Upon degradation, it was possible to observe a clear degradation band of roughly 30 kDa in SDS-PAGE (Figure 5A). An aliquot of fully degraded EcAII was analyzed also by native-PAGE in order to determine if the tetrameric fold was maintained upon degradation. As showed in Figure 5B, the fully degraded protein (lane 2) ran in native conditions as a single species and had reduced molecular weight with regard to the freshly purified protein (lane 3). Western blot analysis (Figure 5C) showed that the fully degraded protein (lane 2) was not reactive to the anti-His tag antibody directed against the protein N-terminus 6xHis tag. Absence of proteases was confirmed by a qualitative test that uses casein as a substrate and Folin-Ciocalteu reagent as a colorimetric indicator (data not shown).

### 8. X-ray structure determination

Single prismatic crystals of the N24S mutant were obtained in 100 mM HEPES, pH 7.5, 5% w/v PEG 8000, 4% v/v ethylene glycol at 21°C after 4 days. The condition resulted to be highly reproducible and efficient in producing crystals of good dimensions and well diffracting. The crystal described in the present paper diffracted at a resolution of 1.51 Å. The crystal asymmetric unit (ASU) contained 2 distinct intimate dimers (namely, A1/C1 and A2/C2); clear electron density corresponding to the L-Asp product was present in all the 4 active sites of the ASU. Two of the 4 monomers (i.e., A1 and C2) presented clear electron density for residues belonging to the active site flexible loop (residues 3-27), while residues 15-26 were missing in monomers A2 and C1.

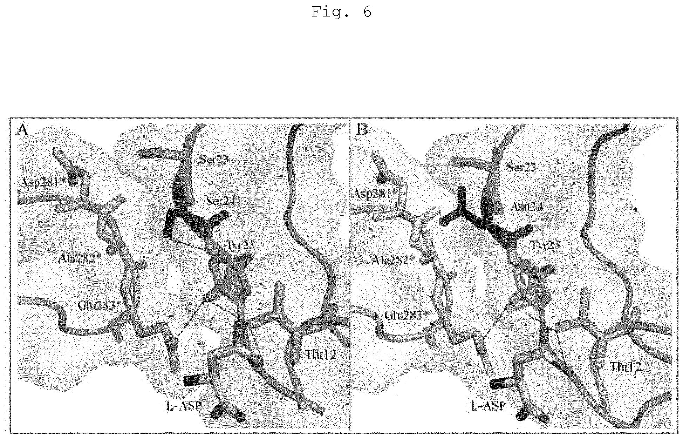

Replacement of the asparagine in position 24 with a serine causes a local H-bonds rearrangement. Particularly, in the mutant protein the H-bond connecting residue Tyr25 with the Ala282 of the nearby monomer is lost in favour of a new H-bond between Tyr25 N and Ser24 Oγ (Figure 6).

Replacement of asparagine 24 with a serine prevents protease activity, with a result similar to the one described for other mutants in the same position. Moreover, removal of the asparagine side chain is likely to prevent spontaneous cleavage of the peptide bond next to asparagine in the presence of water, which is a common undesirable reaction in stored proteins as described by [21].

From the above description, the advantages offered by the present invention will be immediately evident to the person skilled in the art.

In particular, the disclosed asparaginase has shown to preserve the functional parameters compared to the wild-type protein already known.

Accordingly, the preserved catalytic and anti-proliferative properties of the protein, accounting for an improved long-term cytotoxicity, represent a solid basis for its *in vivo* efficacy.

At the same time, however, the protein of the invention has a much improved long-term stability in culture and upon storage, which represents a promising feature for the development and introduction of the drug in the therapy.

In view of the collected data it can be concluded that the protein has IC₅₀ values that are at least equal or even better than the wild-type.

In addition to the above, the disclosed protein is expected to produce less tumor resistance according to *in vitro* data.

The latter feature is of pivotal importance, because the proteases resistance represents a huge improvement in the drug characteristics, in view of the fact that tumor-related proteases may to a large extent impair the efficacy of ASNase by degrading it and by unmasking new immunogenic epitopes, thus increasing the risk of antibody production and relapse.

Studies have revealed that an increased proteolytic activity may be due not only to the tumor mass, but also to the cells surrounding the tumor.

An increased proteolytic activity, which largely contributes to cancerogenesis and tumor metastatization, has been observed in solid tumors.

The present invention also encompasses the possibility of modifying the protein with PEGylation.

Said chemical modification allows for a further improvement in the N24S mutant features, such as in particular, the stability, including the thermal stability, and the reduced immunogenicity, which, in turn, result in an enhanced safety and efficacy of the drug.

As an alternative approach, the present invention also encompasses the possibility of using the GRASPA technology for loading the protein of the invention by encapsulation into red blood cells.

It is known that asparagine reacts with reducing sugars contained in starch-based foods during processing like baking of frying or heating above 120° C and is thus responsible for the production of acrylamide as a product of a Maillard reaction (see, for instance, [22, 23].

Concerns about dietary exposure to acrylamide had arisen as a result of studies conducted in Sweden in 2002, which showed that high levels of acrylamide were formed during the frying or baking of a variety of foods. The Joint FAO/WHO Expert Committee on Food Additives (JECFA) reviewed the safety of acrylamide in 2005 and recommended that acrylamide be re-evaluated when results of ongoing carcinogenicity and long term neurotoxicity studies, which are being conducted around the world, become available and that appropriate efforts to reduce acrylamide concentrations in food should continue.

Accordingly, the present invention finds additional useful application also in the food industry.

In particular, the enzyme of the invention advantageously reduces the time required to inactivate the acrylamide compared to the other known enzymes.

### References

1. Maggi, M. and Chiarelli L. R. and Valentini G. and Scotti C., Engineering of Helicobacter pylori L-asparaginase: characterization of two functionally distinct groups of mutants. PLoS One, 2015. 10(2): p. e0117025.
2. Kidd, J. G., Regression of transplanted lymphomas induced in vivo by means of normal guinea pig serum. II. Studies on the nature of the active serum constituent: histological mechanism of the regression: tests for effects of guinea pig serum on lymphoma cells in vitro. J Exp Med, 1953. 98(6): p. 583--606.
3. Covini, Daniele and Maggi Maristella and Tardito Saverio and Bussolati Ovidio and Chiarelli Laurent R. and Pasquetto Maria V. and Vecchia Luca and Valentini Giovanna and Scotti Claudia, Expanding Targets for a Metabolic Therapy of Cancer: L-Asparaginase. Topics in anti-cancer research, 2015. 3: p. 418--445.
4. Chan, W. K., Lorenzi, P. L., Anishkin, A., Purwaha, P., Rogers, D. M., Sukharev, S., Rempe, S. B., and Weinstein, J. N., The glutaminase activity of L-asparaginase is not required for anticancer activity against ASNS-negative cells. Blood, 2014. 123(23): p. 3596-606.
5. Parmentier, J. H., Maggi, M., Tarasco, E., Scotti, C., Avramis, V. I., and Mittelman, S. D., Glutaminase activity determines cytotoxicity of L-asparaginases on most leukemia cell lines. Leuk Res, 2015. 39(7): p. 757-62.
6. Avramis, V. I., Is glutamine depletion needed in ALL disease? Blood, 2014. 123(23): p. 3532-3.
7. Patel, N., Krishnan, S., Offman, M. N., Krol, M., Moss, C. X., Leighton, C., van Delft, F. W., Holland, M., Liu, J., Alexander, S., Dempsey, C., Ariffin, H., Essink, M., Eden, T. O., Watts, C., Bates, P. A., and Saha, V., A dyad of lymphoblastic lysosomal cysteine proteases degrades the antileukemic drug L-asparaginase. J Clin Invest, 2009. 119(7): p. 1964-73.
8. Fu, Cecilia H. and Sakamoto Kathleen M., PEG-asparaginase. Expert opinion on pharmacotherapy, 2007. 8(12): p. 1977--84.
9. Kwon, Y. M., Chung, H. S., Moon, C., Yockman, J., Park, Y. J., Gitlin, S. D., David, A. E., and Yang, V. C., L-Asparaginase encapsulated intact erythrocytes for treatment of acute lymphoblastic leukemia (ALL). J Control Release, 2009. 139(3): p. 182-9.
10. Godfrin, Y., Thomas, X., Bertrand, Y., and Duget, C., L-Asparaginase Loaded into Erythrocytes (GRASPA): Principle and Interests in Acute Lymphoblastic Leukemia. Blood, 2007. 110(11): p. 4325.
11. Jennings, M. P. and Beacham, I. R., Analysis of the Escherichia coli gene encoding L-asparaginase II, ansB, and its regulation by cyclic AMP receptor and FNR proteins. J Bacteriol, 1990. 172(3): p. 1491-8.
12. Studier, F. W., Protein production by autoinduction in high density shaking cultures. Protein Expr Purif, 2005. 41(1): p. 207-34.
13. Ehsanipour, E. A., Sheng, X., Behan, J. W., Wang, X., Butturini, A., Avramis, V. I., and Mittelman, S. D., Adipocytes cause leukemia cell resistance to L-asparaginase via release of glutamine. Cancer Res, 2013. 73(10): p. 2998-3006. 14. Andreotti, G., Monticelli, M., and Cubellis, M. V., Looking for protein stabilizing drugs with thermal shift assay. Drug Test Anal, 2015. 7 (9) : p. 831-4.
15. ID29: a high-intensity highly automated ESRF beamline for macromolecular crystallography experiments exploiting anomalous scattering. J. Synchrotron Rad. J. Synchrotron Rad, 2012. 19(19): p. 455--461.
16. Battye, T. Geoff G. and Kontogiannis Luke and Johnson Owen and Powell Harold R. and Leslie Andrew G. W., iMOSFLM: a new graphical interface for diffraction-image processing with MOSFLM. Acta crystallographica. Section D, Biological crystallography, 2011. 67(Pt 4): p. 271--81.
17. Evans, Philip, Scaling and assessment of data quality. Acta crystallographica. Section D, Biological crystallography, 2006. 62(Pt 1): p. 72--82.
18. Automating crystallographic structure solution and refinement of protein-ligand complexes. Acta Crystallographica Section D Biological Crystallography, 2014. 70(1): p. 144--154.
19. Swain, A. L., Jaskolski, M., Housset, D., Rao, J. K., and Wlodawer, A., Crystal structure of Escherichia coli L-asparaginase, an enzyme used in cancer therapy. Proc Natl Acad Sci USA, 1993. 90(4): p. 1474-8.
20. Klei, Herbert E. and Moriarty Nigel W. and Echols Nathaniel and Terwilliger Thomas C. and Baldwin Eric T. and Pokross Matt and Posy Shana and Adams Paul D. and IUCr and D. Adams P. and W. Grosse-Kunstleve R. and L. W. Hung and R. Ioerger T. and J. McCoy A. and W. Moriarty N. and J. Read R. and C. Sacchettini J. and K. Sauter N. and C. Terwilliger T. and J. Badger and M. Sauder J. and L. Blundell T. and H. Jhoti and C. Abell and J. Bruno I. and C. Cole J. and M. Kessler and J. Luo and S. Motherwell W. D. and H. Purkis L. and R. Smith B. and R. Taylor and I. Cooper R. and E. Harris S. and G. Orpen A. and E. Carhart R. and H. Smith D. and R. Venkataraghavan and R. Chance M. and P. Cordella L. and P. Foggia and C. Sansone and M. Vento and J. Das and E. Echols and W. Moriarty N. and E. Klei H. and V. Afonine P. and G. Bunkczi and J. Headd J. and J. McCoy A. and R. Oeffner and J. Read R. and C. Terwilliger T. and D. Adams P. and P. Emsley and B. Lohkamp and G. Scott W. and K. Cowtan and C. Finzel B. and R. Akavaram and A. Ragipindi and R. Van Voorst J. and M. Cahn and E. Davis M. and E. Pokross M. and S. Sheriff and T. Baldwin E. and W. Grosse-Kunstleve R. and V. Afonine P. and K. Sauter N. and D. Adams P. and D. Hawkins P. C. and G. Skillman A. and L. Warren G. and A. Ellingson B. and T. Stahl M. and K. Henrick and E. Hopcroft J. and M. Karp R. and E. Klei H. and K. Kish and M. Lin P. F. and Q. Guo and J. Friborg and E. Rose R. and Y. Zhang and V. Goldfarb and R. Langley D. and M. Wittekind and S. Sheriff and J. Kleywegt G. and A. Kumar and H. J. Chiu and L. Axelrod H. and A. Morse and M. A. Elsliger and A. Wilson I. and A. Deacon and A. Lebedev A. and P. Young and N. Isupov M. and V. Moroz O. and A. Vagin A. and N. Murshudov G. and C. Liu and M. Mooij W. T. and J. Hartshorn M. and J. Tickle I. and J. Sharff A. and L. Verdonk M. and H. Jhoti and W. Moriarty N. and W. Grosse-Kunstleve R. and D. Adams P. and J. Oldfield T. and J. Oldfield T. and L. Quan M. and P. Pinto D. J. and A. Rossi K. and S. Sheriff and S. Alexander R. and E. Amparo and K. Kish and M. Knabb R. and M. Luettgen J. and P. Morin and A. Smallwood and J. Woerner F. and R. Wexler R. and J. Rogers D. and T. Tanimoto T. and W. Rose P. and B. Beran and C. Bi and F. Bluhm W. and D. Dimitropoulos and S. Goodsell D. and A. Prlic and M. Quesada and B. Quinn G. and D. Westbrook J. and J. Young and B. Yukich and C. Zardecki and M. Berman H. and E. Bourne P. and W. Schttelkopf A. and F. van Aalten D. M. and C. Terwilliger T. and H. Klei and D. Adams P. and W. Moriarty N. and D. Cohn J. and D. Weininger and S. Wlodek and G. Skillman A. and A. Nicholls, Ligand placement based on prior structures: the guided ligand-replacement method. Acta Crystallographica Section D Biological Crystallography, 2014. 70(1): p. 134--143.
21. Capasso, S., Mazzarella, L., Sorrentino, G., Balboni, G., and Kirby, A. J., Kinetics and mechanism of the cleavage of the peptide bond next to asparagine. Peptides, 1996. 17(6): p. 1075-7.
22. Zyzak, D. V., Sanders, R. A., Stojanovic, M., Tallmadge, D. H., Eberhart, B. L., Ewald, D. K., Gruber, D. C., Morsch, T. R., Strothers, M. A., Rizzi, G. P., and Villagran, M. D., Acrylamide formation mechanism in heated foods. J Agric Food Chem, 2003. 51(16): p. 4782-7.
23. Mottram, D. S., Wedzicha, B. L., and Dodson, A. T., Acrylamide is formed in the Maillard reaction. Nature, 2002. 419(6906): p. 448-9.

## Claims

1. An isolated peptide having the sequence of SEQ.ID. n. 6.

2. The isolated peptide according to the preceding claim, wherein said sequence further comprises a his-tag at the N-terminal.

3. The isolated peptide of claim 1 being encoded by the nucleotidic sequence of SEQ. ID. n. 5.

4. The isolated nucleotidic sequence having a sequence corresponding to the SEQ.ID. n. 5.

5. A vector comprising the isolated nucleotidic sequence of the preceding claim.

6. A host cell cloned with the vector according to the preceding claim.

7. A process for the preparation of the isolated peptide of claim 1 comprising the step of culturing *Escherichia coli* cells which have been cloned with a vector of claim 5 in an appropriate medium, followed by the recovery of the extract and purification of the protein.

8. The isolated peptide of claim 1, which is chemically modified by PEGylation.

9. The isolated peptide of claim 1 or 8 for use as a medicament.

10. The isolated peptide of the preceding claim for use as a medicament in the treatment of solid tumors, both primary and secondary.

11. The isolated peptide according to the preceding claim 9 or 10 for use in the treatment of neoplasia selected in the group comprising: gastrointestinal, lung, breast, prostatic, ovarian, liver, kidney, thyroid, nasopharyngeal tumors, sarcomas, lymphomas and multiple myeloma or in the treatment of Acute Lymphoblastic Leukeamia (ALL).

12. The isolated peptide according to claim 9 for use as a medicament for humans or animals.

13. A pharmaceutical preparation comprising the isolated peptide according to claim 1 or 8 and one of more carriers and/or excipients and/or preservatives pharmaceutically acceptable, which may be encapsulated within red blood cells.

14. The pharmaceutical preparation according to the preceding claim further comprising a chemotherapeutic drug.

15. The pharmaceutical preparation according to the preceding claim, wherein said chemotherapeutic drug is selected in the group comprising: aminoglutethimide, amsacrine, anastrozole, asparaginase, bcg, bicalutamide, bleomycin, bortezomib, buserelin, busulfan, campothecin, capecitabine, carboplatin, carfilzomib, carmustine, chlorambucil, chloroquine, cisplatin, cladribine, clodronate, colchicine, cyclophosphamide, cyproterone, cytarabine, dacarbazine, dactinomycin, daunorubicin, demethoxyviridin, dichloroacetate, dienestrol, diethylstilbestrol, docetaxel, doxorubicin, epirubicin, estradiol, estramustine, etoposide, everolimus, exemestane, filgrastim, fludarabine, fludrocortisone, fluorouracil, fluoxymesterone, flutamide, gemcitabine, genistein, goserelin, hydroxyurea, idarubicin, ifosfamide, imatinib, interferon, irinotecan, ironotecan, lenalidomide, letrozole, leucovorin, leuprolide, levamisole, lomustine, lonidamine, mechlorethamine, medroxyprogesterone, megestrol, melphalan, mercaptopurine, mesna, metformin, methotrexate, mitomycin, mitotane, mitoxantrone, nilutamide, nocodazole, octreotide, oxaliplatin, paclitaxel, pamidronate, pemetrexed, pentostatin, perifosine, plicamycin, pomalidomide, porfimer, procarbazine, raltitrexed, rituximab, sorafenib, streptozocin, sunitinib, suramin, tamoxifen, temozolomide, temsirolimus, teniposide, testosterone, thalidomide, thioguanine, thiotepa, titanocene dichloride, topotecan, trastuzumab, tretinoin, vinblastine, vincristine, vindesine, or vinorelbine.

## Patentansprüche

1. Isoliertes Peptid, welches die Sequenz aus SEQ. ID. n. 6 aufweist.

2. Isoliertes Peptid nach dem vorhergehenden Anspruch, wobei die Sequenz ferner einen His-Tag am N-Terminus umfasst.

3. Isoliertes Peptid aus Anspruch 1, kodiert von der nukleotidischen Sequenz aus SEQ. ID. n. 5.

4. Isolierte nukleotidische Sequenz, welche eine Sequenz entsprechend der SEQ. ID. n. 5 aufweist.

5. Vektor, umfassend die isolierte nukleotidische Sequenz nach dem vorhergehenden Anspruch.

6. Wirtszelle, kloniert mit dem Vektor nach dem vorhergehenden Anspruch.

7. Verfahren zur Herstellung des isolierten Peptids aus Anspruch 1, umfassend den Schritt des Kultivierens von *Escherichia coli* Zellen, welche mit einem Vektor aus Anspruch 5 kloniert wurden, in einem geeigneten Medium, gefolgt von der Gewinnung des Extrakts und Aufreinigung des Proteins.

8. Isoliertes Peptid aus Anspruch 1, welches chemisch modifiziert ist durch PEGylierung.

9. Isoliertes Peptid aus Anspruch 1 oder 8 zur Verwendung als ein Medikament.

10. Isoliertes Peptid nach dem vorhergehenden Anspruch zur Verwendung als ein Medikament in der Behandlung von soliden Tumoren, sowohl primär als auch sekundär.

11. Isoliertes Peptid nach dem vorhergehenden Anspruch 9 oder 10 zur Verwendung in der Behandlung von Tumoren, ausgewählt aus der Gruppe umfassend: Magen-Darm-, Lungen-, Brust-, Prostata-, Eierstock-, Leber-, Nieren-, Schilddrüsen-, Nasopharynx-Tumoren, Sarkomen, Lymphomen und multiplem Myelom oder in der Behandlung akuter lymphatischer Leukämie (ALL).

12. Isoliertes Peptid nach Anspruch 9 zur Verwendung als ein Medikament für Menschen und Tiere.

13. Pharmazeutische Zusammensetzung umfassend das isolierte Peptid nach Anspruch 1 oder 8 und einen von mehreren Trägern und/oder Hilfsstoffen und/oder Konservierungsstoffen, die pharmazeutisch verträglich sind, welche innerhalb roter Blutzellen eingekapselt sein können.

14. Pharmazeutische Zusammensetzung nach dem vorhergehenden Anspruch, ferner umfassend ein chemotherapeutisches Medikament.

15. Pharmazeutische Zusammensetzung nach dem vorhergehenden Anspruch, wobei das chemotherapeutische Medikament ausgewählt ist aus der Gruppe umfassend: Aminoglutethimid, Amsacrin, Anastrozol, Asparaginase, Bcg, Bicalutamid, Bleomycin, Bortezomib, Buserelin, Busulfan, Campothecin, Capecitabin, Carboplatin, Carfilzomib, Carmustin, Chlorambucil, Chloroquin, Cisplatin, Cladribin, Clodronat, Colchicin, Cyclophosphamid, Cyproteron, Cytarabin, Dacarbazin, Dactinomycin, Daunorubicin, Demethoxyviridin, Dichloroacetat, Dienestrol, Diethylstilbestrol, Docetaxel, Doxorubicin, Epirubicin, Estradiol, Estramustin, Etoposid, Everolimus, Exemestan, Filgrastim, Fludarabin, Fludrocortison, Fluoruracil, Fluoxymesteron, Flutamid, Gemcitabin, Genistein, Goserelin, Hydroxyharnstoff, Idarubicin, Ifosfamid, Imatinib, Interferon, Irinotecan, Ironotecan, Lenalidomid, Letrozol, Leucovorin, Leuprolid, Levamisol, Lomustin, Lonidamin, Mechlorethamin, Medroxyprogestron, Megestrol, Melphalan, Mercaptopurin, Mesna, Metformin, Methotrexat, Mitomycin, Mitotan, Mitoxantron, Nilutamid, Nocodazol, Octreotid, Oxaliplatin, Paclitaxel, Pamidronat, Pemetrexed, Pentostatin, Perifosin, Plicamycin, Pomalidomid, Porfimer, Procarbazin, Raltitrexed, Rituximab, Sorafenib, Streptozocin, Sunitinib, Suramin, Tamoxifen, Temozolomid, Temsirolimus, Teniposid, Testosteron, Thalidomid, Thioguanin, Thiotepa, Titanocen-Dichlorid, Topotecan, Trastuzumab, Tretionin, Vinblastin, Vincristin, Vindesin oder Vinorelbin.

## Revendications

1. Peptide isolé ayant la séquence de SEQ. ID. n° 6.

2. Peptide isolé selon la revendication précédente, dans lequel ladite séquence comprend en outre une étiquette His au niveau N-terminal.

3. Peptide isolé selon la revendication 1 qui est codé par la séquence nucléotidique de SEQ. ID. n° 5.

4. Séquence nucléotidique isolée ayant une séquence correspondant à la SEQ. ID. n° 5.

5. Vecteur comprenant la séquence nucléotidique isolée selon la revendication précédente.

6. Cellule hôte clonée avec le vecteur selon la revendication précédente.

7. Procédé pour la préparation du peptide isolé selon la revendication 1, comprenant l'étape de mise en culture de cellules *d'Escherichia coli* qui ont été clonées avec un vecteur selon la revendication 5 dans un milieu approprié, suivie de la récupération de l'extrait et de la purification de la protéine.

8. Peptide isolé selon la revendication 1, qui est chimiquement modifié par PEGylation.

9. Peptide isolé selon la revendication 1 ou 8, destiné à être utilisé comme médicament.

10. Peptide isolé selon la revendication précédente, destiné à être utilisé comme médicament dans le traitement de tumeurs solides, tant primaires que secondaires.

11. Peptide isolé selon la revendication 9 ou 10 précédente, destiné à être utilisé dans le traitement d'une néoplasie choisie dans le groupe comprenant : les tumeurs gastro-intestinales, pulmonaires, du sein, prostatiques, des ovaires, du foie, rénales, de la thyroïde, rhino-pharyngiennes, les sarcomes, les lymphomes et le myélome multiple ou dans le traitement d'une Leucémie Aiguë Lymphoblastique (LAL).

12. Peptide isolé selon la revendication 9, destiné à être utilisé comme médicament pour les humains ou les animaux.

13. Préparation pharmaceutique comprenant le peptide isolé selon la revendication 1 ou 8 et un ou plusieurs véhicules et/ou excipients et/ou conservateurs pharmaceutiquement acceptables, qui peuvent être encapsulés dans des globules rouges.

14. Préparation pharmaceutique selon la revendication précédente, comprenant en outre un médicament chimiothérapeutique.

15. Préparation pharmaceutique selon la revendication précédente, dans laquelle ledit médicament chimiothérapeutique est choisi dans le groupe comprenant les suivants : aminoglutéthimide, amsacrine, anastrozole, asparaginase, bcg, bicalutamide, bléomycine, bortézomib, buséréline, busulfan, campothécine, capécitabine, carboplatine, carfilzomib, carmustine, chlorambucile, chloroquine, cisplatine, cladribine, clodronate, colchicine, cyclophosphamide, cyprotérone, cytarabine, dacarbazine, dactinomycine, daunorubicine, déméthoxyviridine, dichloroacétate, diénestrol, diéthylstilbestrol, docétaxel, doxorubicine, épirubicine, oestradiol, estramustine, étoposide, évérolimus, exémestane, filgrastim, fludarabine, fludrocortisone, fluorouracile, fluoxymestérone, flutamide, gemcitabine, génistéine, goséréline, hydroxyurée, idarubicine, ifosfamide, imatinib, interféron, irinotécan, ironotécan, lénalidomide, létrozole, leucovorine, leuprolide, lévamisole, lomustine, lonidamine, chlorméthine, médroxyprogestérone, mégestrol, melphalan, mercaptopurine, mesna, metformine, méthotrexate, mitomycine, mitotane, mitoxantrone, nilutamide, nocodazole, octréotide, oxaliplatine, paclitaxel, pamidronate, pemetrexed, pentostatine, perifosine, plicamycine, pomalidomide, porfimère, procarbazine, raltitrexed, rituximab, sorafénib, streptozocine, sunitinib, suramine, tamoxifène, témozolomide, temsirolimus, téniposide, testostérone, thalidomide, thioguanine, thiotépa, dichlorure de titanocène, topotécan, trastuzumab, trétinoïne, vinblastine, vincristine, vindesine ou vinorelbine.
